(19) **Europäisches Patentamt / European Patent Office / Office européen des brevets**

(11) **EP 4 529 850 A1**

(12) **EUROPEAN PATENT APPLICATION**

(43) Date of publication:
**02.04.2025 Bulletin 2025/14**

(21) Application number: **23200479.6**

(22) Date of filing: **28.09.2023**

(51) International Patent Classification (IPC):
**A61B 6/03** (2006.01)   **A61B 6/00** (2024.01)
**G16H 50/20** (2018.01)   **A61B 6/46** (2024.01)
**G06T 7/00** (2017.01)

(52) Cooperative Patent Classification (CPC):
**A61B 6/032; A61B 6/488; A61B 6/5247;
A61B 6/544; G06T 3/14; G16H 50/20;** A61B 6/468;
A61B 6/5294; G06T 7/0012

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(71) Applicant: **Koninklijke Philips N.V.
5656 AG Eindhoven (NL)**

(72) Inventors:
• **LUNDT, Bernd**
  **Eindhoven (NL)**
• **MAY, Jan Marek**
  **Eindhoven (NL)**
• **KOEPNICK, Johannes**
  **5656AG Eindhoven (NL)**

(74) Representative: **Philips Intellectual Property &
Standards
High Tech Campus 52
5656 AG Eindhoven (NL)**

(54) **A METHOD FOR THE RETROSPECTIVE FEEDBACK OF THE EFFECTIVE PATIENT DOSE IN RADIOGRAPHIC IMAGING**

(57)     The present invention relates to radiographic imaging. In order to improve the effective dose estimation, there is provided a computer-implemented method (400) for determining an effective dose to a subject from a radiographic examination, in which a radiographic imaging device with a radiation source and a radiation detector is used to acquire a radiographic image of the subject, the method comprising:

a) providing (410) a three-dimensional, 3D, patient model;

b) determining (420) a set of non-rigid registration parameters for an adaption of the 3D patient model to the subject;

c) registering (430) the 3D patient model to the subject using the set of determined non-rigid registration parameters; and

d) determining (440) the effective dose based on the registered 3D patient model of the body part, geometry data of the radiographic imaging device and generator data of the radiation source.

Fig. 5

**Description**

FIELD OF THE INVENTION

The present invention relates to radiographic imaging, and in particular to a computer-implemented method and apparatus for determining an effective dose to a subject from a radiographic examination, to a radiographic imaging system, to a computer program product, and to a computer-readable storage medium.

BACKGROUND OF THE INVENTION

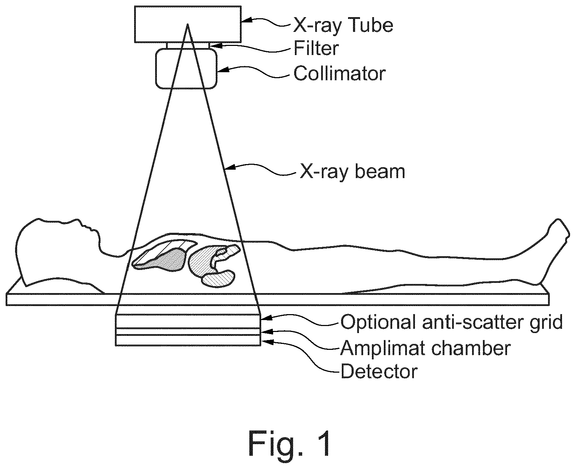

Radiography is an imaging technique using X-rays, gamma rays, or similar ionizing radiation and non-ionizing radiation to view the internal form of an object. Applications of radiography include medical. Fig. 1 illustrates an exemplary X-ray examination on a table. To create an image in the radiography, a beam of X-rays is produced by an X-ray generator and is projected toward a subject. A certain amount of the X-rays or other radiation is absorbed by the subject, dependent on the subject's density and structural composition. The X-rays that pass through the subject are captured behind the subject by an X-ray detector, which may be either photographic film or a digital detector.
Nowadays, the determination of the effective dose is based on rough estimates. One problem may be overlapping organs, as shown in Fig. 1, which may have different sensitivities to X-ray. The thickness, location, and to some extend the shape of the organs may also vary from patient to patient.

SUMMARY OF THE INVENTION

There may be a need to improve the effective dose estimation.
According to a first aspect of the present invention, there is provided a computer-implemented method for determining an effective dose to a subject from a radiographic examination, in which a radiographic imaging device with a radiation source and a radiation detector is used to acquire a radiographic image of the subject, the method comprising:

a) providing a three-dimensional, 3D, patient model, wherein the 3D patient model comprises annotated data with tissue information;
b) determining a set of non-rigid registration parameters for an adaption of the 3D patient model to the subject ;
c) registering the 3D patient model to the subject using the set of determined non-rigid registration parameters; and
d) determining the effective dose based on the registered 3D patient model, geometry data of the radiographic imaging device, and generator data of the radiation source.

Accordingly, the present disclosure proposes to adapt a 3D patient model to a particular patient by using a set of non-rigid registration parameters. The 3D patient model may be a generic representation of a surface of a human or part of a human, e.g., chest. The 3D patient model may be not specific to any patient or is specific to a patient meeting a norm. The 3D patient model includes one or more internal organs and/or one or more tissues. The 3D patient model is an annotated 3D model with annotated data comprising tissue information, which is used to obtain a radiation specific weighting factor and a tissue specific weighting factor of at least one internal organ and/or at least one type of tissues within the 3D patient model. In some examples, the tissue information may comprise an organ identifier used to identify at least one organ within the 3D patient model and/or a tissue identifier used to identify a tissue type of at least one within the 3D patient model. For example, if the internal organ (e.g., liver) comprises a single type of tissues (e.g., liver), the organ identifier may be used to retrieve the tissue specific absorption coefficients and weighting factors from a database for the calculation of a local effective dose, In some examples, the tissue information may comprise the absorption coefficient and the tissue weighting factors. This may be beneficial if the internal organ (e.g., bone marrow) comprise two or more types of tissues. In such case, the tissue information may comprise an attenuation coefficient of at least one tissue type of tissues and a tissue specific weighting factor of at least one tissue type of the tissues within the 3D patient model. From this adapted 3D patient model, the tissue specific absorption coefficients and weighting factors may be taken to determine a local effective dose.
This can be used among others to determine one or more of the following valuable information:
The effective dose of the complete irradiated area, which may be logged in e.g., an X-ray logbook to collect information about the applied effective dose over time. This may be compared with the limits set by local regulatory authorities.
The local effective dose, e.g., within specific organs, which can be logged in an X-ray logbook to collect information about the applied effective dose over time. This can be compared with the limits set by local regulatory authorities.
Together with a software which calculates the optimal collimation it is possible to check whether tissue was unnecessarily irradiated. In this case, this information can be used to give the radiographer feedback not only according to the unnecessarily irradiated area (which is state of the art nowadays) but also according the unnecessarily applied effective dose which is significantly more relevant for the patient.

According to an exemplary embodiment of the first aspect of the present invention, a data-driven model is applied to determine the set of non-rigid registration parameters, wherein the data-driven model has been trained to determine the set of non-rigid registration parameters for a model-to-patient registration using the acquired radiographic image of the subject, and depth image data of the subject.

In one example, the data-driven model may include a neural network model, such as a fully connected neural network, convolutional neural network, or another neural network.

According to an exemplary embodiment of the first aspect of the present invention, the data-driven model comprises a neural network.

According to an exemplary embodiment of the first aspect of the present invention, the 3D patient model is generated based on one or more Computed Tomography, CT, datasets or one or more Magnetic Resonance, MR, datasets.

The one or more CT datasets may comprise CT image data acquired from one or more patients. The one or more MR datasets may comprise MR image data acquired from one or more patients.

According to an exemplary embodiment of the first aspect of the present invention, the tissue information comprises an organ identifier used to identify at least one organ within the 3D patient model and/or a tissue identifier used to identify a tissue type of at least one tissue within the 3D patient model.

The organ identifier may be the name of the at least one internal organ (e.g., kidney) or any other identifier (e.g., code) used to identify the at least one organ within the 3D patient model.

The tissue identifier may be the name of the tissue type or any other identifier (e.g., code) used to identify the tissue type of the at least one tissue within the 3D patient model.

The organ identifier and/or the tissue identifier may be used to retrieve various information from a database. For example, the organ identifier and/or the tissue identifier may be used to retrieve a radiation specific weighting factor and a tissue specific weighting factor of the at least one organ and/ the at least one tissue from the database. Such information may be used to determine a local effective dose.

According to an exemplary embodiment of the first aspect of the present invention, the tissue information comprises one or more of:

- an attenuation coefficient of a tissue type of at least one tissue within the 3D patient model; and
- a tissue specific weighting factor of a tissue type of at least one tissue within the 3D patient model.

In one example, the internal organ may comprise two or more different types of tissues. For example, a long bone which consists among others of cortical bone and spongy bone tissue which have different attenuation coefficients and different tissue specific weighting factors. In such case, it may be beneficial to include the attenuation coefficients and tissue specific weighting factors of different tissue types into the tissue information.

According to an exemplary embodiment of the first aspect of the present invention, the effective dose comprises one or more of:

- an effective dose per volume element;
- an effective dose per organ; and
- an effective dose of a total irradiated area.

According to an exemplary embodiment of the first aspect of the present invention, the method further comprising:

providing one or more of the following information:

- an effective dose of the total irradiated area;
- an effective dose of a specific organ;
- information from one or more previous radiographic examinations;
- an accumulated total and/or an organ-specific dose over a specific time period; and
- a warning signal if the accumulated total and/or the organ-specific dose reaches a predefined threshold.

According to an exemplary embodiment of the first aspect of the present invention, the body part comprises one or more of: at least one organ and/or at least one tissue.

According to an exemplary embodiment of the first aspect of the present invention, the radiation source is configured to emit X-rays, or gamma rays.

According to a second aspect of the present invention, there is provided an apparatus comprising a processor configured to perform the steps according to the first aspect and any associated example.

According to a third aspect of the present invention, there is provided a radiographic imaging system. The radiographic imaging system comprises a radiographic imaging device comprising a radiation source and a radiation detector configured to acquire a radiographic image of a subject, a depth camera configured to acquire a depth image of the subject, and the apparatus according to the second aspect to determine an effective dose to the subject in radiographic imaging.

According to an exemplary embodiment of the third aspect of the present invention, the radiographic imaging device comprises one or more of:

- an X-ray imaging device; and
- a Gamma ray imaging.

According to a further aspect of the present invention, there is provided a computer program product comprising instructions which, when the program is executed by a processor, cause the processor to carry out the steps of the method according to the first aspect and any associated example.

According to another aspect of the present invention, there is provided a computer-readable storage medium having stored thereon the computer program product.

These and other aspects of the present invention will become apparent from and be elucidated with reference to the embodiments described hereinafter.

BRIEF DESCRIPTION OF THE DRAWINGS

These and other aspects of the invention will be apparent from and elucidated further with reference to the embodiments described by way of examples in the following description and with reference to the accompanying drawings, in which

Fig. 1 illustrates an X-ray examination on a table.
Fig. 2 illustrates an exemplary radiographic imaging device.
Fig. 3 illustrates a flow chart diagram of an exemplary training method.
Fig. 4 illustrates a principal geometry which can be used for the calculations for a single voxel.
Fig. 5 illustrates a flow chart diagram of an exemplary computer-implemented method for determining an effective dose to a subject from a radiographic examination.

It should be noted that the figures are purely diagrammatic and not drawn to scale. In the figures, elements which correspond to elements already described may have the same reference numerals. Examples, embodiments or optional features, whether indicated as non-limiting or not, are not to be understood as limiting the invention as claimed.

DETAILED DESCRIPTION OF EMBODIMENTS

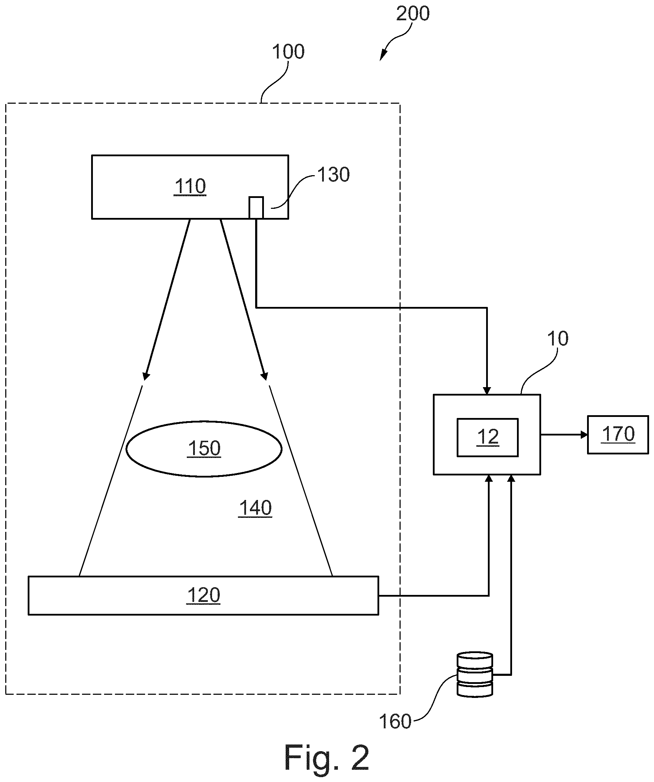

Fig. 2 illustrates an exemplary radiographic imaging system 200. The radiographic imaging system 200 comprises a radiographic imaging device 100 and an effective dose determination apparatus 10.

In the illustrated example, an X-ray imaging device 100 is provided as an example of the radiographic imaging device. While reference is made to the X-ray imaging device 100 in the following description, it will be appreciated that the device, method, and system disclosed herein could also be applied to other radiographic imaging modality, such as imaging using gamma rays.

The X-ray imaging device 100 comprises an X-ray source 110 and an X-ray detector 120 configured to acquire X-ray image data of a subject 150. The X-ray source 110 and the X-ray detector 120 are separated by an examination region 140 for performing an X-ray imaging operation on the subject 150 when the subject is located within the examination region. The solid lines extending between the X-ray source 110 and the X-ray detector 120 indicate the volumetric extent of the overlap between the X-ray beam emitted by the X-ray source 110, and the X-ray radiation-sensitive region of the X-ray detector 120, within which the X-ray image data may be generated. The volumetric extent of this overlap defines the examination region 140. The subject may for example be a human body or a portion of the human body. In one example, the X-ray source is mounted to the ceiling via a gantry, and the X-ray detector is mounted to a stand and held in the vertical position. Alternative arrangements, mounting arrangements, and positions of the X-ray source 110 and the X-ray detector 120 may also be used.

The depth camera 130 is configured to view the examination region 140 in order to generate depth camera image data representing the subject 150 when the subject is located within the examination region 140. The depth camera image data may therefore represent a three-dimensional shape of a surface of the subject 150. In the illustrated example, the depth camera 130 is arranged within a housing of the X-ray source 110. However, the depth camera 130 may alternatively be positioned elsewhere in order to view the examination region 140. The depth camera may be arranged on a wall, or on a ceiling of a room in which the X-ray imaging device 100 is located, or it may be mechanically coupled to a floor-based stand in the room. The depth camera 130 may alternatively be mobile. Examples of the depth camera 130 may include, but are not limited to, a time-of-flight (TOF) camera, a structured-light camera, and a stereo vision camera. The TOF camera generates depth camera image data representing the range of the objects by using the time taken for emitted light pulses to travel from the position of the camera to objects in a scene and back again. In the structured-light camera, an optical pattern is projected on to the surface of the objects within a scene, and the disparity between the original projected pattern and the pattern that is deformed by the surface of the objects is imaged by one or more cameras. In the stereo vision camera, different views of a scene are used to compute a depth map of the scene.

The effective dose determination apparatus 10 comprises a processor 12. The effective dose determination apparatus 10 may be implemented in numerous ways (e.g., such as with dedicated hardware) to perform the functions described herein. A "processor" is one example of the effective dose determination apparatus 10, which employs one or more microprocessors that may be programmed using software (e.g., microcode) to perform various functions described herein. The effective dose determination apparatus 10 may be implemented with or without employing a processor, and also may be implemented as a combination of dedicated hardware to perform some functions and a processor (e.g., one or more programmed microprocessors and associated circuitry) to perform other functions. Examples of device components that may be employed in various embodiments of the present disclosure include, but are not limited to, conventional microprocessors, application specific integrated circuits (ASICs), and field-programmable gate arrays (FPGAs). In various implementations, a processor may be associated with one or more storage media (generically referred to herein as "memory," e.g., volatile and non-volatile computer memory). In some implementations, the storage media may be encoded with one or more programs that, when executed on one or more processors and/or controllers, perform at least some of the functions described herein. Various storage media may be fixed within the effective dose determination apparatus 10 or may be transportable, such that the one or more programs stored thereon can be loaded into the effective dose determination apparatus 10 so as to implement various aspects of the present disclosure described herein. The terms "program" or "computer program" are used herein in a generic sense to refer to any type of computer code (e.g., software or microcode) that can be employed to program one or more processors.

The effective dose determination apparatus 10 is configured to receive the depth image data from the depth camera 130 and the X-ray image data from the X-ray imaging device 100. The effective dose determination apparatus 10 may receive the depth camera image data and the X-ray image data via any form of digital communication. The communication path may be direct, or indirect. For example, the effective dose determination apparatus 10 may be connected to the depth camera 130 and the X-ray detector 120 via a direct wired or wireless communication path, such as an electrical cable, or a wireless infrared or RF communication path such as Bluetooth, as illustrated by the arrows connecting these items in Fig. 2. Alternatively, the communication path may be indirect, and the effective dose determination apparatus 10 may in communication with the depth camera 130 and the X-ray imaging device 100 via the internet, the cloud, or a computer readable storage memory.

To determine an effective dose, the effective dose determination apparatus 10 is configured to receive a 3D patient model. The 3D patient model includes one or more internal organs and/or one or more tissues. The 3D patient model may have been created from 3-dimensional data like CT or MR data. These datasets may be segmented into organ and tissue types. The 3D patient model may be a generic representation of a surface of a human or part of a human, e.g., chest. Different models may be used for different body types, such as a male or female model. The 3D patient model may be not specific to the patient. For example, the 3D patient model may be a statistical shape model. The 3D patient model may be not specific to any patient or is specific to a patient meeting a norm. Any representation may be used for the model. In one example, the 3D patient model may be formed from a mesh, such as a mesh or triangles. Other meshes may be used. Other representations of a 3D surface may be used. In one example, the 3D patient model may further comprise annotated data with tissue information. The tissue information is used to obtain a radiation specific weighting factor and a tissue specific weighting factor of at least one internal organ and/or at least one type of tissues within the 3D patient model. In some examples, the tissue information may comprise an organ identifier used to identify at least one organ within the 3D patient model and/or a tissue identifier used to identify a tissue type of at least one within the 3D patient model. For example, if the internal organ (e.g., liver) comprises a single type of tissues (e.g., liver), the organ identifier may be used to retrieve the tissue specific absorption coefficients and weighting factors from a database for the calculation of a local effective dose, In some examples, the tissue information may comprise the absorption coefficient and the tissue weighting factors. This may be beneficial if the internal organ (e.g., bone marrow) comprise two or more types of tissues. In such case, the tissue information may comprise an attenuation coefficient of at least one tissue type of tissues and a tissue specific weighting factor of at least one tissue type of the tissues within the 3D patient model. From this adapted 3D patient model, the tissue specific absorption coefficients and weighting factors may be taken to determine a local effective dose. The attenuation coefficient is a measure of how much the incident energy beam (e.g., X-rays) is weakened by the material it is passing through, and is dependent on the spectrum of the radiation. The tissue specific weighting factor is a relative measure of the risk of stochastic effects that might result from irradiation of that specific tissue. It accounts for the variable radiation sensitivities of organs and tissues in the body to ionizing radiation. In one example, the effective dose determination apparatus 10 may receive the 3D patient model from a database 160 shown in Fig. 2, which may store a plurality of 3D patient models representing a surface of two or more different body parts (e.g., chest, leg, etc.) of a human.

The effective dose determination apparatus 10 is further configured to determine a set of non-rigid registration parameters for an adaption of the 3D patient model to the subject. As an example, the effective dose determination apparatus 10 may apply a data-driven model to determine the set of non-rigid registration parameters. The data-driven model has been trained to determine the set of non-rigid registration parameters for a model-to-patient registration using the depth camera image data received from the depth camera 130 and the X-ray image data received from the X-ray imaging device 100. The data-driven model may include a suitable algorithm that is trained on the basis of appropriate training data. The data-driven model may include a neural network model, such as a fully connected neural network, convolutional neural network, or another neural network.

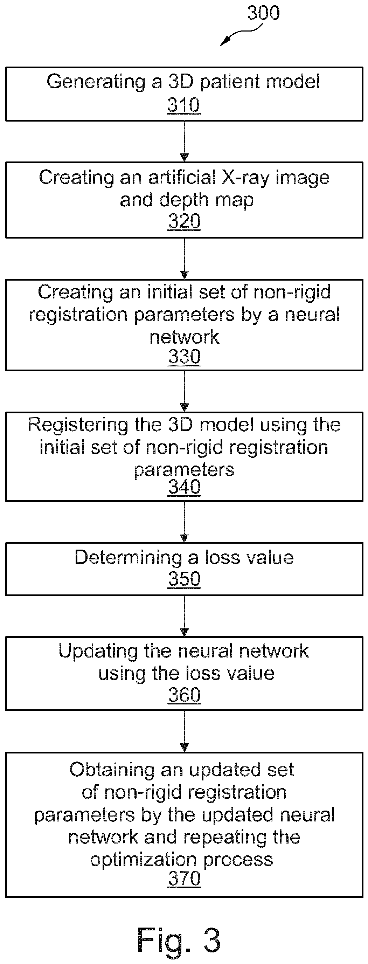

Fig. 3 illustrates a flow chart diagram of an exemplary method 300 for training a neural network. At block 310, a 3D patient model is generated, e.g., based on one or more CT datasets and/or one or more MR datasets. As described above, the 3D patient model may be a generic representation of a surface of a human or part of a human. The 3D patient model may be not specific to the patient. For example, the 3D patient model may be a statistical shape model. The 3D patient model may be not specific to any patient or is specific to a patient meeting a norm. The 3D patient model is an annotated 3D patient model. The annotated data may comprise one or more of the following tissue information, information about one or more organs and/or tissues within the 3D patient model, an attenuation coefficient of a tissue type of at least one tissue within the 3D patient model, and a tissue specific weighting factor of a tissue type of at least one tissue within the 3D patient model.

At block 320, an artificial X-ray image and a depth map are created using one or more CT datasets. This artificial X-ray image and depth map are also referred to as target X-ray image and depth map. For example, the artificial X-ray image may be obtained by converting 3D CT image data into a 2D projection. The depth map comprises information representing a three-dimensional shape of a surface of a body part of a patient. The depth map may be created by contouring an outer surface of one or more CT images.

At block 330, an initial set of non-rigid registration parameters created by a neural network is selected. For training the neural network, the neural network model arrangement or architecture may be defined. The definition may be by configuration or programming of the learning. The number of layers or units, type of learning, and other characteristics of the network are controlled by the programmer or user. In some examples, one or more aspects (e.g., number of nodes, number of layers or units, or type of learning) are defined and selected by the machine during the learning.

At block 340, the 3D patient model is registered using the initial set of non-rigid registration parameters created by the neural network to create an X-ray image and a depth map.

At block 350, a loss value is determined using the difference between the X-ray image and the depth map created from the 3D patient model and the target X-ray image and depth map.

At block 360, the loss value is used to update the neural network.

At block 370, the updated neural network is used to obtain a set of updated registration parameters, and the optimization is repeated until a sufficient quality is reached. The sufficient quality may be indicated by a predefined loss value.

Turning back to Fig. 2, the effective dose determination apparatus 10 is further configured to register the 3D patient model to the subject using the set of determined non-rigid registration parameters, and to determine the effective dose based on the registered 3D patient model, geometry data of the radiographic imaging device, and generator data of the radiation source. The effective dose may include one or more of an effective dose per volume element, an effective dose per organ, and a total effective dose.

The geometry data of the X-ray imaging device 100 may be determined in different ways.

In some examples, the positions of at least one of the X-ray source 110 and the X-ray detector 120 may be fixed positions. For example, the positions of the X-ray source 110 and the X-ray detector may be mechanically attaching the relevant item to a reference position, such as the wall, the ceiling, the floor, and so forth. In these examples, the geometry data of the X-ray imaging device 100 may be determined using calibration data that represents the fixed position(s). In some examples, the positions of both the X-ray source 110 and the X-ray detector 120 are fixed. In these examples, the spatial relationship between the X-ray source 110 and the X-ray detector 120 may be determined based on calibration data representing the relative positions of the X-ray source 110 and the X-ray detector 120.

In some examples, the positions of one or both of the X-ray source 110 and the X-ray detector 120 may be movable to any position e.g., along rails. In these examples, the spatial relationship between the X-ray source 110 and the X-ray detector 120 may be determined using a position sensor. Examples of suitable position sensors may include, but are not limited to, laser-based optical rangefinders, radiofrequency, and ultrasound ranging transponders, and optical cameras that are configured to track the positions of fiducial markers disposed on one or more of the X-ray source 110 and the X-ray detector 120.

The X-ray generator provides operator control of the radiographic techniques, including tube voltage (kVp), tube current (mA), and exposure duration, and delivers power to the x-ray tube. The generator data of the X-ray source 110 may include one or more of the above-mentioned parameters. As an example, the generator data of the X-ray source 110 may comprise the X-ray source spectra and the tube current. The generator data of the X-ray source 110 may be obtained in several ways. In some examples, the generator data of the X-ray source 110 may be obtained from an operating console of the X-ray imaging device 100. In some examples, the generator data of the X-ray source 110 may be provided by a user input. In some examples, the generator data of the X-ray source 110 may be provided by a sensor device, e.g., an X-ray tube current measuring sensor. In some examples, the generator data of the X-ray source may be obtained using two or more of the above-described approaches.

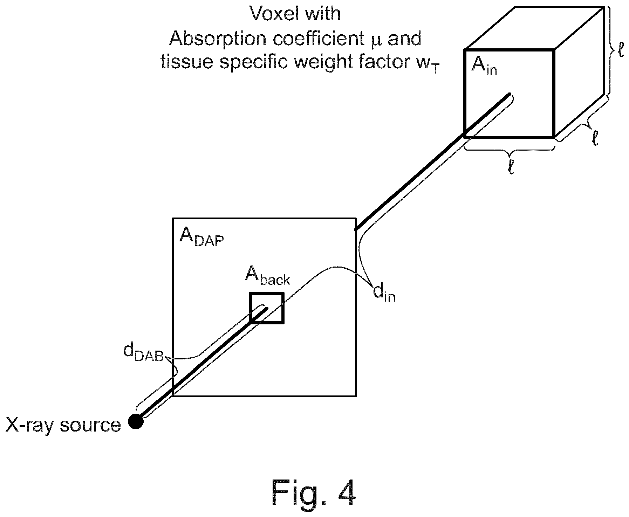

There are various approaches to determine the effective dose. In one example, the determination of the effective dose may be simplified using cubic voxels and no scatter. In practice, minor deviations due to non-cubic voxels and scatter may be considered. Fig. 4 shows a principal geometry which can be used for the calculations for a single voxel. The proportions within the figure are not correct. The intention is to introduce the relevant quantities for the later calculations, which are presented afterwards.

It may be assumed that the dose area product $DAP$ for an area $A_{DAP}$ at a distance $d_{DAP}$ from the X-ray source is known. This means that the total dose $D_{Total}$ irradiated over the complete area can be calculated as:

$$D_{Total} = DAP/A_{DAP}$$

For any subarea $A_{Sub}$ of $A_{DAP}$ ($A_{back}$ is a special case of such a subarea), the irradiated dose $D_{Sub}$ at a distance $d_{DAP}$ from the X-ray source can be calculated as:

$$D_{Sub} = D_{Total} * \frac{A_{Sub}}{A_{DAP}} = \frac{DAP}{A_{DAP}^2} * A_{Sub}$$

Let's assume that the X-Ray beam goes into the z-direction and the entrance area of the voxel is the x-y-plane of the voxel and has a distance $d_{in}$ from the X-ray source. Let's also assume that we have cubic voxels with an edge length $l$.

The entrance area $A_{in}$ of the voxel has the size $l^2$. The back projected entrance area $A_{back}$ (back projected to distance $d_{DAP}$) can be calculated as:

$$A_{back} = A_{in} * \frac{d_{DAP}^2}{d_{in}^2} = l^2 * d_{DAP}^2/d_{in}^2$$

In case of a surface voxel of the patient model, which is from the perspective of the X-ray tube not obscured by other objects, the entrance dose can be calculated as:

$$D_{in} = DAP/A_{DAP}^2 * l^2 * d_{DAP}^2/d_{in}^2$$

Using the absorption coefficient $\mu$ of the tissue within the voxel the exit dose of the voxel can be calculated as:

$$D_{out} = D_{in} * e^{-\mu l}$$

The difference between entrance dose and exit dose is the absorbed Dose $D_{abs}$ within this voxel:

$$D_{abs} = D_{in} - D_{out} = D_{in} * \left(1 - e^{-\mu l}\right) = \frac{DAP}{A_{DAP}^2} * \frac{d_{DAP}^2}{d_{in}^2} * l^2 * \left(1 - e^{-\mu l}\right)$$

On the other hand, is the exit dose $D_{out}$ the input dose of the next voxel at z-position $d_{in} + l$. Iteration over all voxel along the beam delivers the absorbed dose for all voxels along the beam.

Using the radiation specific weight factor $w_R$, which is 1 for X-ray, the equivalent dose per voxel H can now be calculated as follows:

$$H = w_R * D_{abs}$$

Using the tissue specific weight factor $w_T$, which is defined by international or local authorities, the effective dose per voxel E can be calculates as follows:

$$E = w_T * H = w_T * w_R * D_{abs}$$

The total effective dose or the effective dose per organ can now be calculated by summing up the effective dose of all voxels respectively for all voxels belonging to a specific organ.

The results may be stored e.g., in a data storage and/or presented to a user e.g., via a display. For example, as shown in Fig. 2, a display 170 may be configured to receive the results from the effective dose determination apparatus 10 via any form of digital communication. The communication path may be direct, or indirect. For example, the display 170 may be connected to the effective dose determination apparatus 10 via a direct wired or wireless communication path, such as an electrical cable, or a wireless infrared or RF communication path such as Bluetooth, as illustrated by the arrows connecting these items in Fig. 2. Alternatively, the communication path may be indirect, and the effective dose determination apparatus 10 may be in communication with the display 170 via the internet, the cloud, or a computer readable storage memory. The display 170 may be any appropriate display, such as a display on a mobile device, a display of a personal computer, etc.

On the display 170, a graphic user interface (GUI) may be provided to present one or more of the following results to a user. In some examples, the total effective dose may be shown on the display, optionally together with recommended values for the examination or limits set by local regulatory authorities. In some examples, overlays may show selected organs together with the effective dose for this organ. In some examples, together with information from old examination, the accumulated total or organ specific dose over a specific time period can be shown. In some examples, a warning may be shown if the accumulated dose reaches a critical level.

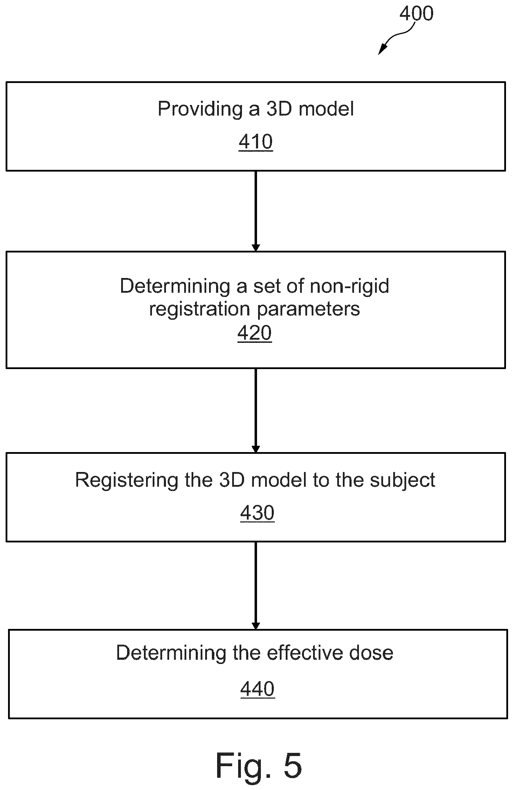

Fig. 5 illustrates a flow chart diagram of an exemplary computer-implemented method 400 for determining an effective dose to a subject from a radiographic examination, in which a radiographic imaging device with a radiation source and a radiation detector is used to acquire a radiographic image of the subject. For example, the radiographic imaging device may be an X-ray imaging device shown in Fig. 2.

At block 410, the method 400 comprises the step of providing a 3D patient model. The 3D patient model may have been created from 3-dimensional data like CT or MR data. These datasets may be segmented into organ and tissue types. The 3D patient model may be a generic representation of a surface of a human or part of a human. Different models may be used for different body types, such as a male or female model. The 3D patient model may be not specific to the patient. For example, the 3D patient model may be a statistical shape model. The 3D patient model may be not specific to any patient or is specific to a patient meeting a norm. Any representation may be used for the model. In one example, the 3D patient model may be formed from a mesh, such as a mesh or triangles. Other meshes may be used. Other representations of a 3D surface may be used. In one example, the 3D patient model may further comprise annotated data with tissue information. Examples of the tissue information may include, but are not limited to, information about one or more organs and/or tissues within 3D patient model, an attenuation coefficient of a tissue type of at least one tissue within the 3D patient model, and a tissue specific weighting factor of a tissue type of at least one tissue within the 3D patient model. The attenuation coefficient is a measure of how much the incident energy beam (e.g., X-rays) is weakened by the material it is passing through, and is dependent on the spectrum of the radiation. The tissue specific weighting factor is a relative measure of the risk of stochastic effects that might result from irradiation of that specific tissue. In one example, the 3D patient model may be provided by a database 160 shown in Fig. 2, which may store a plurality of 3D patient models of different body parts.

At block 420, the method 400 comprises the step of determining a set of non-rigid registration parameters for an adaption of the 3D patient model to the subject. For example, a data-driven model may be applied, which has been trained to determine the set of non-rigid registration parameters for a model-to-patient registration using the depth camera image data and the X-ray image data. The data-driven model may include a suitable algorithm that is trained on the basis of appropriate training data. In one example, the data-driven model may include a neural network model, such as a fully connected neural network, convolutional neural network, or another neural network. An exemplary training method is described in detail hereinbefore and in particular with respect to the flow chart diagram shown in Fig. 3.

At block 430, the method 400 comprises the step of registering the 3D patient model to the subject using the set of determined non-rigid registration parameters.

At block 440, the method 400 comprises the step of determining the effective dose based on the registered 3D patient model and one or more system dependent parameters of the radiation imaging device. The determination of the effective dose may be simplified using cubic voxels and no scatter. In practice, minor deviations due to non-cubic voxels and scatter may be considered. Fig. 4 shows the principal geometry which can be used for the calculations for a single voxel.

In another exemplary embodiment of the present invention, a computer program or a computer program element is provided that is characterized by being adapted to execute the method steps of the method according to one of the preceding embodiments, on an appropriate system.

The computer program element might therefore be stored on a computer unit, which might also be part of an embodiment of the present invention. This computing unit may be adapted to perform or induce a performing of the steps of the method described above. Moreover, it may be adapted to operate the components of the above described apparatus. The computing unit can be adapted to operate automatically and/or to execute the orders of a user. A computer program may be loaded into a working memory of a data processor. The data processor may thus be equipped to carry out the method of the invention.

This exemplary embodiment of the invention covers both, a computer program that right from the beginning uses the invention and a computer program that by means of an up-date turns an existing program into a program that uses the invention.

Further on, the computer program element might be able to provide all necessary steps to fulfil the procedure of an exemplary embodiment of the method as described above.

According to a further exemplary embodiment of the present invention, a computer readable medium, such as a CD-ROM, is presented wherein the computer readable medium has a computer program element stored on it which computer program element is described by the preceding section.

A computer program may be stored and/or distributed on a suitable medium, such as an optical storage medium or a solid state medium supplied together with or as part of other hardware, but may also be distributed in other forms, such as via the internet or other wired or wireless telecommunication systems.

However, the computer program may also be presented over a network like the World Wide Web and can be downloaded into the working memory of a data processor from such a network. According to a further exemplary embodiment of the present invention, a medium for making a computer program element available for downloading is provided, which computer program element is arranged to perform a method according to one of the previously described embodiments of the invention.

It has to be noted that embodiments of the invention are described with reference to different subject matters. In particular, some embodiments are described with reference to method type claims whereas other embodiments are described with reference to the device type claims. However, a person skilled in the art will gather from the above and the following description that, unless otherwise notified, in addition to any combination of features belonging to one type of subject matter also any combination between features relating to different subject matters is considered to be disclosed with this application. However, all features can be combined providing synergetic effects that are more than the simple summation of the features.

While the invention has been illustrated and described in detail in the drawings and foregoing description, such illustration and description are to be considered illustrative or exemplary and not restrictive. The invention is not limited to the disclosed embodiments. Other variations to the disclosed embodiments can be understood and effected by those skilled in the art in practicing a claimed invention, from a study of the drawings, the disclosure, and the dependent claims.

In the claims, the word "comprising" does not exclude other elements or steps, and the indefinite article "a" or "an" does not exclude a plurality. A single processor or other unit may fulfil the functions of several items re-cited in the claims. The mere fact that certain measures are re-cited in mutually different dependent claims does not indicate that a combination of these measures cannot be used to advantage. Any reference signs in the claims should not be construed as limiting the scope.

## Claims

1. A computer-implemented method (400) for determining an effective dose to a subject from a radiographic examination, in which a radiographic imaging device with a radiation source and a radiation detector is used to acquire a radiographic image of the subject, the method comprising:

   a) providing (410) a three-dimensional, 3D, patient model, wherein the 3D patient model comprises annotated data including tissue information;
   b) determining (420) a set of non-rigid registration parameters for an adaption of the 3D patient model to the subject;
   c) registering (430) the 3D patient model to the subject using the set of determined non-rigid registration parameters; and
   d) determining (440) the effective dose based on the registered 3D patient model, geometry data of the radiographic imaging device, and generator data of the radiation source.

2. The computer-implemented method according to claim 1,
   wherein a data-driven model is applied to determine the set of non-rigid registration parameters, wherein the data-driven model has been trained to determine the set of non-rigid registration parameters for a model-to-patient registration using the acquired radiographic image of the subject, and depth image data of the subject.

**3.** The computer-implemented method according to claim 2,
wherein the data-driven model comprises a neural network.

**4.** The computer-implemented method according to any one of the preceding claims,
wherein the 3D patient model is generated based on one or more Computed Tomography, CT, datasets or one or more Magnetic Resonance, MR, datasets acquired from one or more subjects.

**5.** The computer-implemented method according to any one of the preceding claims,
wherein the tissue information comprises an organ identifier used to identify at least one organ within the 3D patient model and/or a tissue identifier used to identify a tissue type of at least one tissue within the 3D patient model.

**6.** The computer-implemented method according to any one of the preceding claims,
wherein the annotated data further comprises one or more of:

- an attenuation coefficient of a tissue type of at least one tissue within the 3D patient model; and
- a tissue specific weighting factor of a tissue type of at least one tissue within the 3D patient model.

**7.** The computer-implemented method according to any one of the preceding claims,
wherein the effective dose comprises one or more of:

- an effective dose per volume element;
- an effective dose per organ; and
- an effective dose of a total irradiated area.

**8.** The computer-implemented method according to any one of the preceding claims, further comprising:
providing one or more of the following information:

- an effective dose of the total irradiated area;
- an effective dose of a specific organ;
- information from one or more previous radiographic examinations;
- an accumulated total and/or an organ-specific dose over a specific time period; and
- a warning signal if the accumulated total and/or the organ-specific dose reaches a predefined threshold.

**9.** The computer-implemented method according to any one of the preceding claims,
wherein the 3D patient model comprises one or more of: at least one organ and/or at least one tissue.

**10.** The computer-implemented method according to any one of the preceding claims,
wherein the radiation source is configured to emit X-rays or gamma rays.

**11.** An effective dose determination apparatus (10) comprising a processor (12) configured to perform the steps according to any one of the preceding claims.

**12.** A radiographic imaging system (200), comprising:

- a radiographic imaging device (100) comprising a radiation source (110) and a radiation detector (120) configured to acquire a radiographic image of a subject;
- a depth camera (130) configured to acquire depth image data of the subject; and
- the apparatus according to claim 11 to determine an effective dose to the subject in radiographic imaging.

**13.** The radiographic imaging system according to claim 12,
wherein the radiographic imaging device comprises one or more of:

- an X-ray imaging device; and
- a Gamma ray imaging.

**14.** A computer program product comprising instructions which, when the program is executed by a processor, cause the processor to carry out the steps of the method of any one of claims 1 to 11.

**15.** A computer-readable storage medium having stored thereon the computer program product of claim 14.

X-ray Tube
Filter
Collimator

X-ray beam

Optional anti-scatter grid
Amplimat chamber
Detector

Fig. 1

Fig. 2

300

Generating a 3D patient model
310

Creating an artificial X-ray image
and depth map
320

Creating an initial set of non-rigid
registration parameters by a neural
network
330

Registering the 3D model using the
initial set of non-rigid registration
parameters
340

Determining a loss value
350

Updating the neural network
using the loss value
360

Obtaining an updated set
of non-rigid registration
parameters by the updated neural
network and repeating the
optimization process
370

Fig. 3

Fig. 4

400

```
┌─────────────────────────────────────┐
│        Providing a 3D model          │
│                410                   │
└─────────────────────────────────────┘
                    │
                    ▼
┌─────────────────────────────────────┐
│     Determining a set of non-rigid   │
│       registration parameters        │
│                420                   │
└─────────────────────────────────────┘
                    │
                    ▼
┌─────────────────────────────────────┐
│   Registering the 3D model to the    │
│              subject                 │
│                430                   │
└─────────────────────────────────────┘
                    │
                    ▼
┌─────────────────────────────────────┐
│      Determining the effective dose  │
│                440                   │
└─────────────────────────────────────┘
```

Fig. 5

EP 4 529 850 A1

## EUROPEAN SEARCH REPORT

Europäisches Patentamt
European Patent Office
Office européen des brevets

Application Number

EP 23 20 0479

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2012/148131 A1 (COUCH GREGORY [CA] ET AL) 14 June 2012 (2012-06-14)<br>* paragraph [0002] *<br>* paragraph [0025] – paragraph [0026] *<br>* paragraph [0034] – paragraph [0036] *<br>* paragraph [0047] – paragraph [0050] *<br>* paragraph [0052] – paragraph [0053] *<br>* paragraph [0087] – paragraph [0088] *<br>* paragraph [0093] – paragraph [0094] *<br>* figures 1,4,6,9,10 *<br>_____ | 1-15 | INV.<br>A61B6/03<br>A61B6/00<br>G16H50/20<br><br>ADD.<br>A61B6/46<br>G06T7/00 |
| A | US 2020/000425 A1 (JI MIN [CN] ET AL) 2 January 2020 (2020-01-02)<br>* paragraph [0088] – paragraph [0090] *<br>* claims 1,6 *<br>* figure 5 *<br>_____ | 1-15 | |
| A | US 2023/080631 A1 (WANG ADAM S [US] ET AL) 16 March 2023 (2023-03-16)<br>* paragraph [0046] – paragraph [0048] *<br>* paragraph [0054] *<br>* paragraph [0069] – paragraph [0070] *<br>* paragraph [0073] *<br>* paragraph [0082] *<br>* figures 1,2 *<br>_____<br>-/-- | 1-15 | |

TECHNICAL FIELDS SEARCHED (IPC)

A61B
G06T
G16H

The present search report has been drawn up for all claims

| Place of search | Date of completion of the search | Examiner |
|---|---|---|
| Munich | 23 February 2024 | De la Hera, Germán |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

page 1 of 2

Europäisches
Patentamt

European
Patent Office

Office européen
des brevets

# EUROPEAN SEARCH REPORT

Application Number

EP 23 20 0479

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | LEE MIN SUN ET AL: "Deep-dose: a voxel dose estimation method using deep convolutional neural network for personalized internal dosimetry", SCIENTIFIC REPORTS, vol. 9, no. 1, 16 July 2019 (2019-07-16), XP093108820, US ISSN: 2045-2322, DOI: 10.1038/s41598-019-46620-y Retrieved from the Internet: URL:https://www.nature.com/articles/s41598-019-46620-y> [retrieved on 2019-07-16] * the whole document * | 1-15 | |
| A | MAIER JOSCHA ET AL: "Real-Time Patient-Specific CT Dose Estimation using a Deep Convolutional Neural Network", 2018 IEEE NUCLEAR SCIENCE SYMPOSIUM AND MEDICAL IMAGING CONFERENCE PROCEEDINGS (NSS/MIC), IEEE, 10 November 2018 (2018-11-10), pages 1-3, XP033613047, DOI: 10.1109/NSSMIC.2018.8824626 [retrieved on 2019-09-04] * the whole document * | 1-15 | TECHNICAL FIELDS SEARCHED (IPC) |

The present search report has been drawn up for all claims

| Place of search | Date of completion of the search | Examiner |
|---|---|---|
| Munich | 23 February 2024 | De la Hera, Germán |

CATEGORY OF CITED DOCUMENTS

X : particularly relevant if taken alone
Y : particularly relevant if combined with another
document of the same category
A : technological background
O : non-written disclosure
P : intermediate document

T : theory or principle underlying the invention
E : earlier patent document, but published on, or
after the filing date
D : document cited in the application
L : document cited for other reasons

& : member of the same patent family, corresponding
document

EPO FORM 1503 03.82 (P04C01)

**page 2 of 2**

## ANNEX TO THE EUROPEAN SEARCH REPORT
## ON EUROPEAN PATENT APPLICATION NO.

EP 23 20 0479

This annex lists the patent family members relating to the patent documents cited in the above-mentioned European search report.
The members are as contained in the European Patent Office EDP file on
The European Patent Office is in no way liable for these particulars which are merely given for the purpose of information.

23-02-2024

| Patent document cited in search report | | Publication date | Patent family member(s) | | Publication date |
|---|---|---|---|---|---|
| US 2012148131 | A1 | 14-06-2012 | AU | 2011340078 A1 | 27-06-2013 |
| | | | AU | 2016203814 A1 | 30-06-2016 |
| | | | AU | 2017251773 A1 | 16-11-2017 |
| | | | AU | 2017251775 A1 | 16-11-2017 |
| | | | BR | 112013013804 A2 | 13-09-2016 |
| | | | BR | 122017007260 A2 | 14-06-2012 |
| | | | CA | 2819331 A1 | 14-06-2012 |
| | | | CA | 2993848 A1 | 14-06-2012 |
| | | | CA | 3033428 A1 | 14-06-2012 |
| | | | CN | 103442644 A | 11-12-2013 |
| | | | CN | 107095689 A | 29-08-2017 |
| | | | CN | 113856066 A | 31-12-2021 |
| | | | DK | 2648621 T3 | 22-10-2018 |
| | | | DK | 3150126 T3 | 19-11-2018 |
| | | | DK | 3281586 T3 | 24-06-2019 |
| | | | DK | 3524159 T3 | 06-04-2021 |
| | | | EP | 2648621 A1 | 16-10-2013 |
| | | | EP | 3150126 A1 | 05-04-2017 |
| | | | EP | 3281586 A2 | 14-02-2018 |
| | | | EP | 3524159 A1 | 14-08-2019 |
| | | | ES | 2689751 T3 | 15-11-2018 |
| | | | ES | 2692793 T3 | 05-12-2018 |
| | | | ES | 2733922 T3 | 03-12-2019 |
| | | | ES | 2864040 T3 | 13-10-2021 |
| | | | HK | 1242946 A1 | 06-07-2018 |
| | | | JP | 5917552 B2 | 18-05-2016 |
| | | | JP | 6189468 B2 | 30-08-2017 |
| | | | JP | 6395911 B2 | 26-09-2018 |
| | | | JP | 6417000 B2 | 31-10-2018 |
| | | | JP | 2013544605 A | 19-12-2013 |
| | | | JP | 2016129716 A | 21-07-2016 |
| | | | JP | 2017192808 A | 26-10-2017 |
| | | | JP | 2017192809 A | 26-10-2017 |
| | | | KR | 20130130772 A | 02-12-2013 |
| | | | KR | 20170004029 A | 10-01-2017 |
| | | | KR | 20180037288 A | 11-04-2018 |
| | | | KR | 20190075169 A | 28-06-2019 |
| | | | MX | 347370 B | 25-04-2017 |
| | | | MX | 368991 B | 24-10-2019 |
| | | | NZ | 611554 A | 24-12-2014 |
| | | | RU | 2013127784 A | 20-01-2015 |
| | | | RU | 2016124149 A | 03-12-2018 |
| | | | US | 2012148131 A1 | 14-06-2012 |
| | | | US | 2012148132 A1 | 14-06-2012 |
| | | | US | 2012150505 A1 | 14-06-2012 |
| | | | US | 2015139519 A1 | 21-05-2015 |

EPO FORM P0459

For more details about this annex : see Official Journal of the European Patent Office, No. 12/82

page 1 of 2

**ANNEX TO THE EUROPEAN SEARCH REPORT
ON EUROPEAN PATENT APPLICATION NO.**

EP 23 20 0479

This annex lists the patent family members relating to the patent documents cited in the above-mentioned European search report.
The members are as contained in the European Patent Office EDP file on
The European Patent Office is in no way liable for these particulars which are merely given for the purpose of information.

23-02-2024

| Patent document cited in search report | | Publication date | Patent family member(s) | | Publication date |
|---|---|---|---|---|---|
| | | | US | 2015170363 A1 | 18-06-2015 |
| | | | US | 2017123074 A1 | 04-05-2017 |
| | | | US | 2017228860 A1 | 10-08-2017 |
| | | | US | 2017243350 A1 | 24-08-2017 |
| | | | WO | 2012075577 A1 | 14-06-2012 |
| | | | ZA | 201303796 B | 24-06-2015 |
| | | | ZA | 201409203 B | 27-09-2017 |
| | | | ZA | 201606246 B | 28-07-2021 |
| | | | ZA | 201706370 B | 29-01-2020 |
| US 2020000425 | A1 | 02-01-2020 | US | 2020000425 A1 | 02-01-2020 |
| | | | US | 2021121153 A1 | 29-04-2021 |
| US 2023080631 | A1 | 16-03-2023 | CN | 115797252 A | 14-03-2023 |
| | | | EP | 4147644 A1 | 15-03-2023 |
| | | | US | 2023080631 A1 | 16-03-2023 |
| | | | US | 2023081601 A1 | 16-03-2023 |

EPO FORM P0459

For more details about this annex : see Official Journal of the European Patent Office, No. 12/82

page 2 of 2